# EUROPEAN PATENT SPECIFICATION

(11) **EP 2 512 948 B1**
(45) Date of publication and mention of the grant of the patent: **22.03.2017**
(21) Application number: 10798882.6
(22) Date of filing: 15.12.2010
(51) Int. Cl.: B65D 47/08, A61L 12/08

(54) **A CLOSURE FOR CONTAINERS OF OPHTHALMIC SOLUTIONS**
VERSCHLUSS FÜR BEHÄLTER OPHTHALMISCHER LÖSUNGEN
FERMETURE POUR CONTENANTS DE SOLUTIONS OPHTALMIQUES

(30) Priority: 14.12.2010 US 967822; 16.12.2009 US 286937 P
(43) Date of publication of application: 24.10.2012
(73) Proprietor: Johnson & Johnson Vision Care, Inc., Jacksonville, FL 32256 (US)
(72) Inventor: HUNTINGTON, Elysha, Jersey City, NJ 07302 (US); TANAKA, Richard, Astoria, NY 11106 (US); YUMUL, Anthony, New York, NY 10002 (US)
(74) Representative: Tunstall, Christopher Stephen
(86) International application number: PCT/US2010/060395
(87) International publication number: WO 2011/084445

(56) References cited:
- EP-A1- 0 309 396
- WO-A1-00/54622
- WO-A1-99/59886
- WO-A1-2005/113365
- WO-A2-2005/039660
- US-A- 4 711 360
- US-A- 6 050 434

## Description

### FIELD OF THE INVENTION

This invention relates to designs that are used to close containers of ophthalmic solutions and methods of using the same.

### BACKGROUND

Ophthalmic lenses are extremely popular with consumers, particular the soft contact lenses that are either daily disposable or reusable. There are a variety of solutions that are used provide additional comfort to lens wearers when the lenses are in the eye or when the lenses are removed from the eye for cleaning. Most of these solutions are dispensed to the consumer in multiple use bottles that will be opened and reused over time. Given that these solutions are in contact with the eye, the solutions are sterilized to prevent harmful environmental contaminants such as bacteria, viruses, and the like from infecting the patient. However, with a multiple use bottle, these bottles are opened by the consumer and therefore, the consumer's use of the bottle often introduces such contaminants to the solutions. For example commonly used bottles for contact lens solutions have a short cap which covers the spout from which solutions are dispensed. When consumers open these bottles, their fingers often brush across the spout of the bottle and the material on their hands is a source of contamination for the solutions.

The prior art is also disclosed in documents WO 00/54622 A1, WO 2005/039660 A2, WO 99/59886 A1, EP 0 309 396 A1, and WO 2005/113365 A1.

It would be useful if there
was a closure which could be used that inhibits consumers from touching the spouts of bottles when opening or closing said bottles. This need is met by the following invention.

### BRIEF DESCRIPTION OF THE DRAWINGS

- Fig. 1: Perspective view of a closure of the invention on a bottle
- Fig. 2: Perspective view of the base
- Fig. 3: Perspective view of the inside of the cap
- Fig. 4: Top plan view of the closure on a bottle in the open position
- Fig. 5: Cross-section view of the closure on a bottle in the open position
- Fig. 6: Expanded view of Fig. 5
- Fig. 7: Cross-section view of the closure on a bottle in the closed position
- Fig. 8: Expanded view of Fig. 7
- Fig. 9: Angled closure
- Fig. 10: Angled closure
- Fig. 11: Button closure
- Fig. 12: Button closure

### DETAILED DESCRIPTION OF THE INVENTION

This invention includes a closure according to claim 1 or 2.

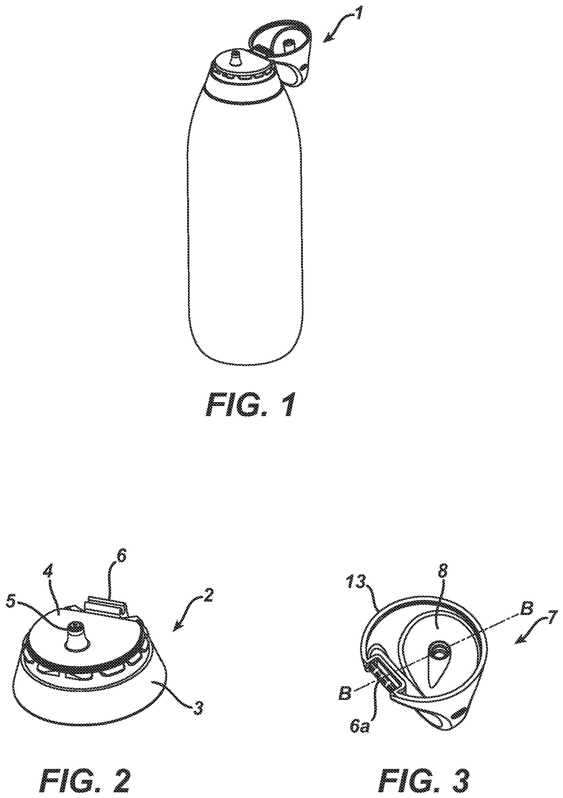

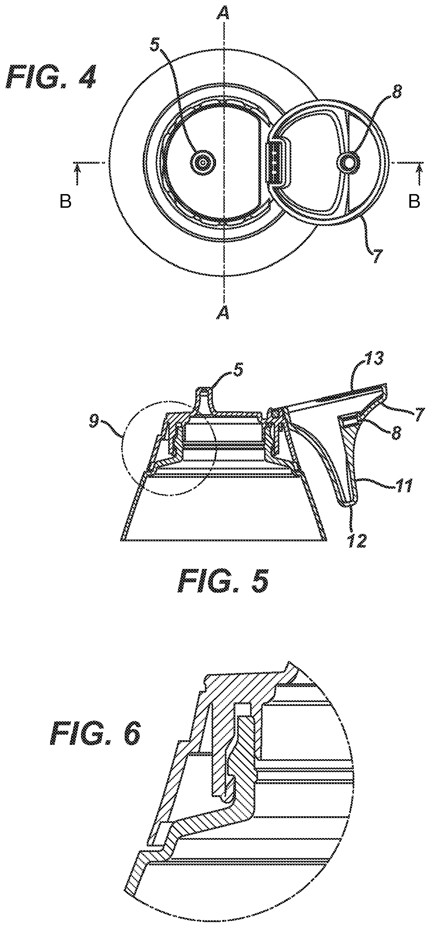

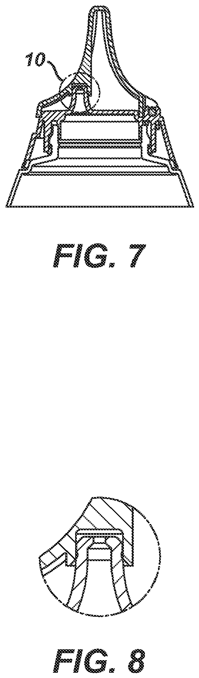

The following figures illustrate an embodiment of the invention. Fig. 1 illustrates a perspective view of a closure 1 of the invention attached to a bottle. Fig. 2 illustrates a perspective view of base 2, neck 3, upper surface 4, and spout 5 and hinge 6. Fig. 3 illustrates cap 7, spout cap 8, and rim 13. The cap may be connected to the base by a number of hinging methods including interlocking hinges and living hinges. Fig. 4 illustrates a top plan view of closure 1 on a bottle in the open position. Spout cap 8 and spout 5 line up along line 5-5. The center of spout 5 is located 5.0 mm from line A-A. Fig. 5 shows a cross-sectional view along line B-B illustrating the interconnection of neck 3 with the neck of the bottle in the circled area 9. Angled exposed front surface 11, is illustrated angling from rim 13 to apex 12. It is preferred that exposed front surface 11 is angled to permit a user's thumb to rest against the such front surface 11 when said bottle is grasped in the same hand. The top of the cap is slightly rounded and apex 12 is the geometric centerpoint of the top of the cap. The height of the cap from rim 13 to apex 12 is 28.42 mm. The partial diameter of rim 13 measured from the circumference of rim 13 to the midpoint of hinge 6 as position 6a, along line B-B is 26.4 mm. Fig. 6 illustrates a more detailed view of circled area 9. Fig. 7 illustrates cross-sectional view along line B-B of closure 1 in the closed position where the mating of spout 5 and spout cap 8 appears in circle 10. Fig. 8 illustrates a more detailed view of circle 10. The mating of this area is a snap fit where spout cap 8 fits over spout 5. However, this mating may be accomplished by a number of methods, including pressure fits.

The foregoing closures may be made by a variety of plastic materials such as without limitation, ethylene vinyl alcohol ("EVA"), fluorinated polymers including without limitation, polytetrafluoroethylene ("PTFE") and polyvinylidene fluoride (" PVDF"), polypropylene, polyethylene, polyisobutylene, nylon, polyurethanes, polyacrylates and methacrylates, polyvinyl palmitate, polyvinyl stearates, polyvinyl myristate, cyanoacrylates, epoxies, silicones, copolymers thereof. The closure is preferably made of polypropylene. Each piece of the closure may be made of a different material or the same material. Any of these pieces may be made of unitary construction with or without the bottle. In the preferred embodiment all pieces of the closure are made of the same material. Any or all of the components of the closure may be made by injection molding (two material injection molding, over-molding, sandwich molding or insert molding). Other combinations of materials and construction methods are known to those of skill in the art of molding plastic materials.

Still further, the invention includes a method according to claim 3.

The aforementioned terms all have their stated meanings and preferred ranges or components. The term "ophthalmic lens solution" means any solution that is used to clean, maintain, or lubricate ophthalmic lenses or the eye of a user of such lenses whether or not such lenses are in the user's eye. Examples of such solutions include any composition which can be directly instilled into an eye, or which can be used to soak, clean, rinse, store or treat any ophthalmic device which can be used placed in or on the eye. Examples of ophthalmic compositions that may be topically administered to the eye, ophthalmic device packing solutions, cleaning solutions, conditioning solutions, storage solutions, eye drops, eye washes, as well as ophthalmic suspensions, aerosols, gels and ointments and the like. In one embodiment of the present invention, the ophthalmic composition is a multipurpose lens care solution. The multipurpose lens care solution may contain a disinfectant. The disinfecting agent should not cause stinging or damage to the eye at use concentrations and should be inert with respect to the other composition components. Suitable disinfecting components include hydrogen peroxide, polymeric biguanides, polymeric quarternary ammonium compounds, chlorites, bisbiguanides, quarternary ammonium compounds and mixtures thereof. The multipurpose lens care solution may also contain one or more lubricating agents may also be included in the ophthalmic composition. Lubricating agents include water soluble cellulosic compounds, hyaluronic acid, and hyaluronic acid derivatives, chitosan, water soluble organic polymers, including water soluble polyurethanes, polyethylene glycols, combinations thereof and the like. Specific examples of suitable lubricating agents include polyvinyl pyrrolidone ("PVP"), hydroxypropyl methyl cellulose, carboxymethyl cellulose, glycerol, propylene glycol, 1,3-propanediol, polyethylene glycols, mixtures there of and the like. The multipurpose lens care solution may also contain one or more surfactant, detergent, or mixtures thereof. Suitable examples include tyloxapol, poloxomer (poly(ethylene oxide)-b-poly(propylene oxide)-b-poly(ethylene oxide)) type surfactants which are commercially available from BASF and poloxamine type surfactants (non-ionic, tetrafunctional block copolymers based on ethylene oxide/propylene oxide, terminating in primary hydroxyl groups, commercially available from BASF, under the tradename Tetronic). A specific example is Pluronic F-147 and Tetronic 1304. Tyloxapol is a non-ionic, low molecular weight surfactant, and is fully soluble in the phosphate buffers. Tyloxapol is a detergent commercially available from Pressure Chemical Company. The multipurpose lens care solution may also contain one or more viscosity adjusting agent or thickener. Suitable viscosity adjusting agents are known in the art and include polyvinyl alcohol, polyethylene glycols, guar gum, combinations thereof and the like. The viscosity adjusting agent may be used in amounts necessary to achieve the desired viscosity. The multipurpose lens care solution may further comprise additional components such as, but not limited to pH adjusting agents, tonicity adjusting agents, buffering agents, active agents, lubricating agents, disinfecting agents, viscosity adjusting agents, surfactants and mixtures thereof. When the ophthalmic composition is an ophthalmic solution, all components in the ophthalmic solution of the present invention should be water soluble. As used herein, water soluble means that the components, either alone or in combination with other components, do not form precipitates or gel particles visible to the human eye at the concentrations selected and across the temperatures and pH regimes common for manufacturing, sterilizing and storing the ophthalmic composition.

The multipurpose lens care solution may also contain one or more active agent. A wide variety of therapeutic agents may be used, so long as the selected active agent is inert in the presence of peroxides. Suitable therapeutic agents include those that treat or target any part of the ocular environment, including the anterior and posterior sections of the eye and include pharmaceutical agents, vitamins, nutraceuticals combinations thereof and the like. Suitable classes of active agents include antihistamines, antibiotics, glaucoma medication, carbonic anhydrase inhibitors, anti-viral agents, anti-inflammatory agents, non-steroid anti-inflammatory drugs, antifungal drugs, anesthetic agents, miotics, mydriatics, immunosuppressive agents, antiparasitic drugs, anti-protozoal drugs, combinations thereof and the like. When active agents are included, they are included in an amount sufficient to produce the desired therapeutic result (a "therapeutically effective amount").

The advantages of the invention are many. For example, users of containers of ophthalmic lens solutions which are closed with the closures of the invention are substantially inhibited from touching the spout of such closures upon opening such containers. This reduces the chance that the consumer will contaminate an ophthalmic solution stored in such containers.

### EXAMPLE 1

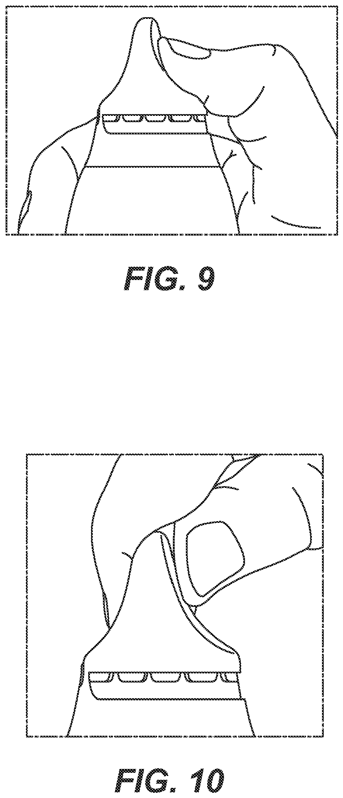

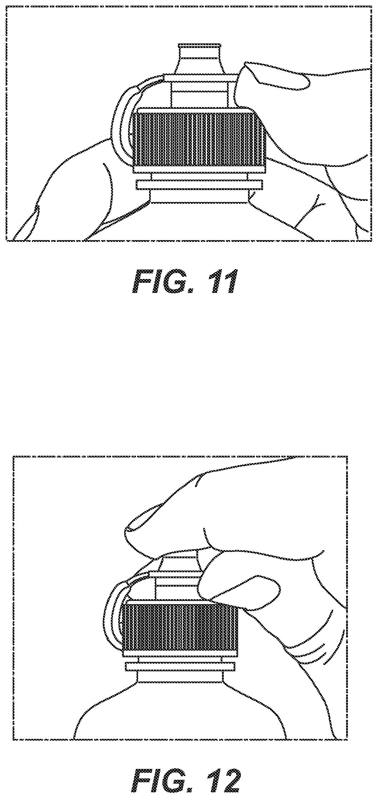

To determine whether bottles closed with the closures of the invention inhibited a user from touching the spout when opening a bottle of contact lens solution, the following test was conducted. The bottles topped with the closures of the invention, Figs. 9 and 10 ("angled closures) were compared to bottles topped with the most common closures, Figs. 11 and 12 (button closures). Figs. 9 and 11 illustrate the "one finger method of opening the bottles and Figs. 10 and 12 illustrate the two fingered approach for opening the bottles. The bottles did not contain any solution, and the spout of each bottle was marked with a UV security pen (Dri-Mark Products Inc.).

A group of contact lens users washed their hands and were shown open bottles with an angled closure and a button closure and the tester closed each of the bottle in front of the subject to illustrate the general operation. The subjects were instructed to follow their normal lens care routine, but not to remove their lenses. Each subject was evaluated visually by the tester to see bottles, to determine if they touched the spout when they opened the bottles. In addition, after opening each bottle, each subject hands were evaluated using a UV light to see if any of the marker was transferred to their hand. Ten of the twelve subjects were observed touching the spout when they opened the button closures. Nine of ten of those finding were confirmed by examining the subjects hands with UV light. With the same twelve subjects opening the angled closure, visual evaluation showed that none of them touched the spout when they opening the angles closure. This finding was confirmed by examining their hands with UV light.

The foregoing embodiments are only meant to illustrate, and not to limit, the invention, which is defined by the appended claims.

## Claims

1. A closure (1) for covering a container for an ophthalmic solution, said closure comprising
a base (2) comprising an upper surface (4) and a neck (3),
wherein said upper surface (4) is attached to said neck (3) and is adapted to sit above an opening of a bottle, and wherein said neck (3) is adapted to be fastened to the opening of the bottle,
wherein said upper surface (4) comprises a spout (5);
a cap (7) comprising an exposed front surface (11), a front inside surface, an apex (12), and a cap rim (13);
wherein the back of said cap (7) is pivotally attached to said base (2) to permit movement of the cap relative to the base between an open position and a closed position,
wherein each of said exposed front surface (11) and said front inside surface comprises a bottom end and a top end,
wherein said bottom end of said exposed front surface (11) and said front inside surface are located closer to the upper surface if the cap (7) is in the closed position and the top end is located further away from the upper surface if the cap (7) is in the closed position,
wherein said front inside surface comprises a spout cap (8) formed between said bottom end and said top end of said front inside surface,
wherein if said cap is closed, the spout cap mates with said spout to form a liquid tight seal,
wherein if said cap is opened, the liquid tight seal between the spout and the spout cap is opened;
**characterized in that**
said exposed front surface (11) comprises an upwardly facing concavity that extends from said cap rim at its bottom end towards said apex at its top end to permit the thumb to rest against the concavity if the bottle is grasped in the same hand, such that the cap can be opened by the thumb without touching the spout.

2. A closure according to claim 1
wherein the front inside surface is angled and the angled front inside surface sits at an angle from the cap rim (13) at its bottom end towards the apex (12) at its top end.

3. A method of storing an ophthalmic lens solution in a bottle, wherein the bottle comprises a closure (1) according to claim 1 or claim 2.

## Patentansprüche

1. Verschluss (1) zum Abdecken eines Behälters für eine ophthalmische Lösung, wobei der Verschluss Folgendes umfasst:
eine Basis (2), die eine obere Fläche (4) und einen Hals (3) umfasst, wobei die obere Fläche (4) an dem Hals (3) angebracht und geeignet ist, über einer Öffnung einer Flasche zu sitzen, und wobei der Hals (3) geeignet ist, an der Öffnung der Flasche befestigt zu werden,
wobei die obere Fläche (4) einen Ausgießer (5) umfasst,
eine Kappe (7), die eine freiliegende vordere Fläche (11), eine vordere Innenfläche, eine Spitze (12) und einen Kappenrand (13) umfasst,
wobei die Rückseite der Kappe (7) schwenkend an der Basis (2) angebracht ist, um eine Bewegung der Kappe bezüglich der Basis zwischen einer offenen Position und einer geschlossenen Position zu gestatten,
wobei die freiliegende vordere Fläche (11) und die vordere Innenfläche jeweils ein unteres Ende und ein oberes Ende umfassen,
wobei das untere Ende der freiliegenden vorderen Fläche (11) und der vorderen Innenfläche näher an der oberen Fläche angeordnet ist, wenn die Kappe (7) in der geschlossenen Position ist, und das obere Ende weiter von der oberen Fläche weg angeordnet ist, wenn die Kappe (7) in der geschlossenen Position ist,
wobei die vordere Innenfläche eine Ausgießerkappe (8) umfasst, die zwischen dem unteren Ende und dem oberen Ende der vorderen Innenfläche ausgebildet ist,
wobei die Ausgießerkappe bei geschlossener Kappe mit dem Ausgießer zusammenpasst, um eine flüssigkeitsdichte Abdichtung zu bilden,
wobei die flüssigkeitsdichte Abdichtung zwischen dem Ausgießer und der Ausgießerkappe bei geöffneter Kappe geöffnet ist,
**dadurch gekennzeichnet, dass**
die freiliegende vordere Fläche (11) eine nach oben weisende Konkavität umfasst, die sich vom Kappenrand an ihrem unteren Ende zu der Spitze an ihrem oberen Ende erstreckt, damit der Daumen an der Konkavität anliegen kann, wenn die Flasche mit derselben Hand ergriffen wird, so dass die Kappe ohne Berührung des Ausgießers mit dem Daumen geöffnet werden kann.

2. Verschluss nach Anspruch 1, wobei die vordere Innenfläche abgewinkelt ist und die abgewinkelte vordere Innenfläche vom Kappenrand (13) an ihrem unteren Ende zur Spitze (12) an ihrem oberen Ende in einem Winkel sitzt.

3. Verfahren zum Aufbewahren einer ophthalmischen Linsenlösung in einer Flasche, wobei die Flasche einen Verschluss (1) nach Anspruch 1 oder Anspruch 2 umfasst.

## Revendications

1. Bouchon (1) pour couvrir un contenant pour une solution ophtalmique, ledit bouchon comprenant une base (2) qui comprend une surface supérieure (4) et un col (3),
dans lequel ladite surface supérieure (4) est fixée audit col (3) et est apte à se trouver au-dessus de l'ouverture d'une bouteille, et dans lequel ledit col (3) est apte à être fixé à l'ouverture de la bouteille,
dans lequel ladite surface supérieure (4) comprend :
un bec verseur (5) ;
un capuchon (7) comprenant une surface avant (11) à découvert, une surface intérieure avant, un apex (12) et un bord (13) de capuchon,
dans lequel le dos dudit capuchon (7) est fixé pivotant à ladite base (2) pour permettre au capuchon de bouger par rapport à la base entre une position ouverte et une position fermée,
dans lequel tant la surface avant (11) à découvert que la surface intérieure avant comprend une extrémité inférieure et une extrémité supérieure,
dans lequel ladite extrémité inférieure de ladite surface avant (11) à découvert et ladite surface intérieure avant sont situées plus près de la surface supérieure si le capuchon (7) est en position fermée et l'extrémité supérieure est situé plus loin de la surface supérieure si le capuchon (7) est en position fermée,
dans lequel ladite surface intérieure avant comprend un capuchon (8) de bec verseur formé entre ladite extrémité inférieure et ladite extrémité supérieure de ladite surface intérieure avant,
dans lequel, si ledit capuchon est fermé, le capuchon de bec verseur s'accouple au bec verseur pour constituer un joint étanche aux liquides,
dans lequel, si ledit capuchon est ouvert, le joint étanche aux liquides entre le bec verseur et le capuchon de bec verseur est ouvert,
**caractérisé en ce que** ladite surface avant (11) à découvert comprend une concavité tournée vers le haut qui s'étend dudit bord de capuchon à son extrémité inférieure vers ledit apex à son extrémité supérieure pour permettre au pouce d'appuyer contre la cavité si on saisit la bouteille dans la même main, de telle sorte qu'on peut ouvrir le capuchon avec le pouce sans toucher le bec verseur.

2. Bouchon selon la revendication 1, dans lequel la surface intérieure avant est inclinée et la surface intérieure avant inclinée forme un angle du bord (13) de capuchon à son extrémité inférieure vers l'apex (12) à son extrémité supérieure.

3. Procédé de stockage d'une solution pour lentilles ophtalmiques dans un flacon, dans lequel le flacon comprend un bouchon (1) selon la revendication 1 ou la revendication 2.
